(19) Europäisches Patentamt
European Patent Office
Office européen des brevets

(11) Publication number: **0 138 553 A2**

(12) **EUROPEAN PATENT APPLICATION**

(21) Application number: **84306867.7**

(22) Date of filing: **09.10.84**

(51) Int. Cl.⁴: **C 07 C 69/24**, C 07 C 67/60

(30) Priority: **11.10.83 US 540724**

(43) Date of publication of application: **24.04.85 Bulletin 85/17**

(84) Designated Contracting States: **AT BE CH DE FR GB IT LI LU NL SE**

(71) Applicant: **ELI LILLY AND COMPANY, 307, East McCarty Street, Indianapolis Indiana 46285 (US)**

(72) Inventor: **Gunn, Bruce Paul, 806 Edgar Allen Poe Lane, Mundelein Illinois 60060 (US)**
Inventor: **Lechleiter, John Clifford, Chatsworth Buckhurst Road, Ascot Berkshire (GB)**

(74) Representative: **Tapping, Kenneth George et al, Lilly Industries Limited Patent Department Erl Wood Manor, Windlesham Surrey, GU20 6PH (GB)**

(54) **Purification of esters of alkanoic acids.**

(57) Alkyl esters of 2-ethyl-2-methylbutanoic acid are purified by selective hydrolysis with an alkali metal hydroxide.

EP 0 138 553 A2

# PURIFICATION OF ESTERS OF ALKANOIC ACIDS

This invention belongs to the field of synthetic organic chemistry and provides a process for purifying esters of a certain highly branched alkanoic acid by selective hydrolysis.

The present invention operates by the hydrolysis of an alkyl ester of an alkanoic acid with a strong alkali metal base, one of the oldest processes in organic chemistry. The unique feature of the present invention is its ability to hydrolyze selectively the esters of certain alkanoic acids, leaving the corresponding ester of the desired acid intact, to be easily separated from the saponified contaminants.

The present invention provides a process for purifying an alkyl ester of the formula

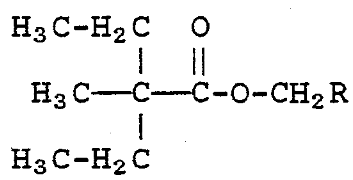

wherein

R is $C_2$-$C_5$ alkyl; provided that each carbon atom in an alkyl R group has at least one hydrogen atom bonded to it;
which ester is mixed with one or more contaminating alkyl esters of the formula

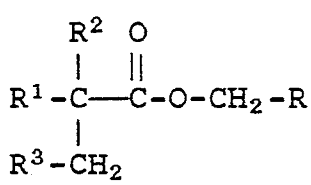

wherein

$R^1$ and $R^2$ are independently hydrogen or methyl;

$R^3$ is $C_1$-$C_5$ alkyl;

which process comprises selectively hydrolyzing the contaminating alkyl ester or esters with an alkali metal hydroxide and separating the resulting alkali metal carboxylate.

All temperatures in the present document are described in degrees Celsius.

The generally named alkyl groups R and $R^3$ used in the above description are described in terms usual in organic chemirstry. $R^3$ may be any alkyl group having not more than five carbon atoms, such as methyl, ethyl, isopropyl, butyl, pentyl, t-butyl, isobutyl, s-butyl, 1-ethylpropyl, 1-methylbutyl, 2,2-dimethylpropyl and the like. The group R may be an alkyl group of from 2 to 5 carbon atoms, but it is limited by the requirement that each carbon atom must have at least 1 hydrogen atom bonded to it. Thus, R may not include groups such as t-butyl or neopentyl, but includes such typical groups as methyl, ethyl, isopropyl, butyl, s-butyl, pentyl, 1,2-dimethylpropyl, 3-methylbutyl, 1-methylbutyl, and 2-methylbutyl.

The preferred esters are $C_3$-$C_5$ alkyl esters, wherein R is $C_2$-$C_4$ alkyl, especially $C_3$-$C_5$ branched alkyl esters, and most especially the isobutyl ester, wherein R is isopropyl.

The contaminating alkyl esters which are separated by the present process include esters of alkanoic acids such as the following typical examples.

2-methylbutanoic acid,
2,2-dimethylpentanoic acid,
2-methylhexanoic acid,
2,4-dimethylpentanoic acid,
heptanoic acid,
2,2,4,4-tetramethylpentanoic acid,
5-methylhexanoic acid,
2-methyloctanoic acid,
4-methylheptanoic acid,
2,2,5,5-tetramethylhexanoic acid,
2,2,4,5-tetramethylhexanoic acid.

The most important contaminating esters are those wherein $R^1$ and $R^2$ are methyl, and $R^3$ is $C_2$-$C_3$ alkyl.

The esters of 2-ethyl-2-methylbutanoic acid, the purification of which is the objective of the present invention, are useful as intermediates for preparing various useful products. Most particularly, they are useful for preparing a series of herbicides which are 5-(2,6-disubstituted-benzamido)-3-(1-ethyl-1-methylpropyl)isoxazoles. Such herbicides have been disclosed in U.S. Patent No. 4,416,683, see Example 1 which shows an alkyl ester of the present tertiary alkanoic acid used as the starting material in the synthesis of a preferred product.

The most preferred mixture of esters on which to carry out the present process is obtained by the esterification of a mixed carboxylic acid product obtained from commercial sources. The product contains a major amount of 2-ethyl-2-methylbutanoic acid, and approximately 30% by weight of contaminating carboxylic acids, of which the major one is 2,2-dimethylpentanoic acid. The mixed acids are esterified by the usual methods, such as by reaction with an appropriate alkanol with sulfuric acid or toluenesulfonic acid as catalyst, and the mixture of esters is used as the starting material for the purposes of the present invention.

The reagent used in the present invention is an alkali metal hydroxide. Sodium hydroxide is preferred but potassium and lithium hydroxides may be used if desired. It is preferred to carry out the hydrolysis in a 2-phase reaction mixture. For example, the mixture of esters may be dissolved in a water-immiscible organic solvent, and mixed with aqueous alkali metal hydroxide. It is most convenient to choose an organic solvent which boils under ambient pressure at the desired operating temperature of the process, so that the hydrolysis may be carried out under r@flux. Obviously, however, the process may be operated under pressure if it is desired to operate above the ambient pressure boiling point of the mixture.

When a 2-phase reaction mixture is used, it is convenient to use an aromatic reaction solvent, especially a xylene or mixed xylenes, or toluene, since the boiling points of those solvents are convenient for the hydrolysis and they are highly resistant to the strong base.

When a water-immiscible organic solvent is used in the reaction mixture, it is most convenient to synthesize the initial mixture of esters in that organic solvent.

When the hydrolysis is over, isolation of the purified product ester is easy when a 2-phase reaction mixture is used. It is necessary only to separate the layers and, if necessary, wash the organic layer with water.

When a 2-phase reaction mixture is used, it is sometimes advantageous to use a phase transfer catalyst in the mixture, particularly when the reaction temperature is comparatively low. The usual types of phase transfer catalysts, such as quarternary ammonium halides, are effective. For example, such phase transfer catalysts as tributylmethylammonium chloride, trimethylphenylammonium iodide, tetrabutylammonium chloride, benzyltriethylammonium methanesulfonate, methylphenyldipropylammonium chloride, butyltrimethylammonium sulfonate and the like may be used as desired

It has been found that the addition of a small amount, in the range of several tenths to several mole percent, preferably from about 1% to about 5% of an alkanoic acid can also serve as a catalyst or initiator in the present process. For example, 2,2-dimethylpentanoic acid has itself been successfully used for this purpose. Numerous other alkanoic acids may be used, such as acetic acid, butyric acid, heptanoic acid, 2-methylpropanoic acid, 3-ethylpentanoic acid, octanoic acid, 2-methylpentanoic acid, for example. The mechanism is not understood, but the fact is quite

useful, especially when a relatively low reaction temperature is used.

If a 1-phase reaction mixture is desired, it is preferred to use an aqueous alkanol, such as ethanol, propanol, butanol, and the like as solvent. The selective hydrolysis which is the basis of the present invention occurs in such a mixture, but the isolation of the purified ester from the saponified contaminants is obviously more difficult, and so operation in a single-phase mixture is not preferred.

The amount of base to be used in the hydrolysis varies from about 1 to about 15 equivalents, based on the total of the mixture of esters submitted to the process. It is more preferred to use from about 1 to about 5 equivalents, and most preferred to use from about 1 to about 2 equivalents under the most highly preferred conditions.

The reaction temperature can be varied between about 60° and about 200°, preferably between about 100° and about 160°. It is obvious that the amount of reagent, the reaction temperature and the reaction time must always be balanced for the best results in any organic chemical process. In the present instance, the product ester is hydrolyzed to some extent under the reaction conditions, although it is selectively hydrolyzed to a lesser extent than are the contaminating esters. Thus, the maximum yield of the desired ester depends on balancing the conditions for the maximum selectivity, i.e., minimal hydrolysis of the desired product. It has been found that it is preferable to operate at a relatively high temperature,

with a relatively small amount of base and for a short reaction time, as the examples reported below demonstrate. The most highly preferred temperature is about 140°, for about 1-2 hours in the presence of about 1-2 equivalents of the alkali metal hydroxide, in a 2-phase reaction mixture.

No phase transfer catalyst or added alkanoic acid is needed in the most highly preferred conditions, but the use of such an additive is useful when the process is operated at lower temperatures in the range of about 60-120°, for example.

However, the examples below also demonstrate that useful purification is obtained at other operating conditions. Reaction times as long as 8 hours have been satisfactorily used, at lower temperatures such as 60-80°, and much larger amounts of base than the most preferred amounts have also successfully been used. It will be understood that the exact optimum operating conditions will vary as different ester mixtures are submitted to the process. Depending on the relative amount of more highly hindered or less highly hindered contaminating esters, more or less stringent reaction conditions will be demanded. Accordingly, the reader must realize that some small amount of experimentation will usually be needed to find the optimum operating conditions for a given feed-stock. The examples below clearly explain the type of experiments which are called for, and it will be seen that the experiments can be planned and carried out by any organic chemist.

The invention is further explained by the following examples. The ultimate starting material for

all of the examples was a commercial C-7 acid mixture containing approximately 70% by weight of 2-ethyl-2-methylbutanoic acid, 26% of 2,2-dimethylpentanoic acid and 4% of 2,2,3-trimethylbutanoic acid. The ratio of the three esters in the ester mixtures used in the examples below was the same as the ratio in the acid mixture, unless otherwise stated.

The following preparation of the mixed esters is typical.

## Preparation 1

### Isobutyl esters of commercial C-7 acid mixture

A 5-liter flask was fitted with a Dean-Stark trap, a condenser and a stirrer. To it were charged 1302 g. of commercial C-7 acid mixture, 815 g. of isobutanol, 133 g. of toluenesulfonic acid and 1000 ml. of xylene. The mixture was heated for about 20 hours while the water formed in the reaction was removed by azeotropic distillation. The mixture was then cooled and the organic layer was separated and washed with 500 ml. of water, with three 300 ml. portions of 10% aqueous sodium carbonate, and again with 500 ml. of water. The washed organic layer was then dried by azeotropic distillation.

The organic layer was analyzed by gas chromatography using an instrument equipped with a capillary injector, a flame ionization detector and a 30-meter x 0.3 mm. OV-101 fused silica capillary column. The injector and detector temperatures were 275°, the flow

rate 2 ml./minute, the split ratio 75:1 and the injection volume 0.5 mcl. The sample concentration was 40 mg./ml. in dichloromethane and the internal standard was 8 mg./ml. dodecane.

The product layer was found to contain 1746 g. of total esters, a yield of 93.7% of the theoretical yield. It contained 45.5% of isobutyl 2-ethyl-2-methylbutanoate, 16.6% of isobutyl 2,2-dimethylpentanoate, 2.81% of 2,3,3-trimethylbutanoate and 35.1% of xylene.

The above analytical method is useful for the hydrolyzed ester product, as well, and was used to analyze most of the following examples.

The first group of examples of purifications according to the present process demonstrate hydrolysis in single-phase reaction systems.

## Example 1

Five g. of isobutyl esters of the commercial C-7 acid mixture was isolated by distillation from an esterification in cyclohexane with sulfuric acid as the catalyst, and was mixed with 4.5 g. of solid potassium hydroxide and 10 ml. of denatured ethanol. The mixture was heated under reflux with stirring, and samples were taken after 5 minutes, 20 minutes, 45 minutes, and 2 hours of treatment. The samples were analyzed and it was found that the ester of 2,2-dimethylpentanoic acid had substantially disappeared after 20 minutes of reflux.

## Example 2

Five g. of mixed isobutyl esters, prepared as described in Example 1, was combined with 10 ml. of 5N sodium hydroxide and 15 ml. of ethanol, and the mixture was stirred under reflux for 4 hours. Analysis of the reaction mixture by chromatography showed that the ratio of the amount of the desired ester to the ester of 2,2-dimethylpentanoic acid was 83/17.

## Example 3

The process of Example 2 was followed again, but for longer periods of time. After 5 hours, the ratio of the desired product to the isobutyl 2,2-dimethylpentanoate was 84.1/15.9, and after 7 hours, the ratio was 90.5/9.5.

## Example 4

The process of Example 2 was carried out again, with a 4 hour reflux time. Chromatographic analysis of the mixture showed that the ratio of the desired ester to the 2,2-dimethylpentan@ate was 85/15; 3.1 g. of the desired ester was found in the reaction mixture.

## Example 5

The process of Example 2 was used again, except that the reflux time was 5 hours. Analysis

identified 3.16 g. of the desired ester in the reaction mixture, together with 0.38 g. of the 2,2-dimethylpentanoate, a ratio of 89.4/10.6.

The following examples report hydrolyses carried out in toluene.

Example 6

A 14.2 g. portion of a toluene solution of isobutyl esters of the commercial C-7 acid mixture, containing 3.53 millimoles of total esters per gram of solution, was combined with 10 ml. of 50% aqueous sodium hydroxide, and the mixture was stirred under reflux for 4 hours. It was then cooled, diluted with 20 ml. of water, and the organic layer was separated. The aqueous layer was extracted with a small amount of toluene and the organic layer was combined with the first organic layer. Chromatographic analysis of the combined organics showed that the product contained 5.25 g. of the desired ester, a yield of 78.6%; the ratio of the desired ester to the 2,2-dimethylpentanoate was 95/5.

Example 7

Seventeen g. of a toluene solution of isobutyl esters of the commercial C-7 acid mixture, containing 2.94 millimoles of total esters per gram, was combined with 10 ml. of 15% aqueous sodium hydroxide, and was stirred under reflux for 4 hours. The mixture was worked up as described in Example 6 and the toluene

solution was analyzed. The ratio of desired ester to 2,2-dimethylpentanoate was 93.7/6.3, and the weight recovery of the desired ester was 80%.

Example 8

The process of Example 7 was carried out twice more, once for 6 hours of reflux time and once for 9 hours. Analysis of the 6-hour run indicated that the weight recovery of the desired ester was 72%, and the ratio of the amount of the desired ester to the 2,2-dimethylpentanoate was 98.3/1.7. At 9 hours, the recovery of the desired ester was 51%.

Example 9

The process of Example 6 was repeated, except that samples were taken from the refluxing reaction mixture at various time intervals. Each sample was about 1 ml. of the organic phase, which was washed with 2 ml. of water, dried over sodium sulfate and analyzed. At 1.5 hours, the ratio of the amount of the desired ester to the 2,2-dimethylpentanoate was 78.6/21.4; at 4 hours it was 98.7/1.3; at 7 hours there was no detectable dimethylpentanoate present.

Example 10

A 14.2 g. portion of the toluene solution of isobutyl esters from Example 6 was combined with 20 ml. of 5N sodium hydroxide and 30 ml. of denatured ethanol,

and the mixture was stirred under reflux, under a nitrogen blanket, for 8 hours. It was a 2-phase mixture. The mixture was then cooled, the phases were separated, and the aqueous phase was washed with 15 ml. of toluene. The organic phases were combined and washed with 15 ml. of water, and the solution was dried over sodium sulfate. Chromatographic analysis indicated that the ratio of the amount of the desired ester to the dimethylpentanoate was 83.9/16.1, and 85.8% of the desired ester was recovered.

Example 11

A 51 g. portion of a toluene solution of isobutyl esters of the commercial C-7 acid mixture, containing 2.94 millimoles of total esters per gram of solution, was added to 30 ml. of 50% aqueous sodium hydroxide, and the mixture was stirred under reflux for 4 hours. A similar mixture was run for 6 hours. At the end of the reflux times, the mixtures were cooled and diluted with water, and the phases were separated. The aqueous layer was extracted with toluene, and the combined organics were dried over sodium sulfate and analyzed chromatographically. The product solution from the 4-hour run contained the desired ester and the 2,2-dimethylpentanoate in a ratio of 94.9/5.1, and the recovery of the desired ester was 76.9%. The product solution from the 6-hour run contained the same substances in a ratio of 95.2/4.7, and the recovery of the desired ester was 64.8%.

## Examples 12-15

Each example was carried out by combining 102.9 g. of a toluene solution of isobutyl esters of commercial C-7 acid mixture, containing 2.43 millimoles of total esters per gram, and in which the ratio of the desired ester to the 2,2-dimethylpentanoate was 61.5/38.5, with 65 ml. of 50% aqueous sodium hydroxide. Various reaction times and temperatures were used. A 4.2 g. portion of tetrabutylammonium hydrogen sulfate was added as a phase transfer catalyst. At the end of the reaction time, the mixtures were cooled, and the organic layer was removed and washed twice with 30 ml. portions of water and once with saturated aqueous sodium chloride. The organic layer was then dried over sodium sulfate and analyzed chromatographically. The results were shown in the table below as the ratio of the desired ester to the dimethylpentanoate.

| Example | Temperature | Time | Product |
|---|---|---|---|
| 12 | Reflux | 7 hr. | 100/0 |
| 13 | Reflux | 2 hr. | 81/19 |
| 14 | Reflux | 7 hr. | 98/2 |
| 15 | 60° | 7 hr. | 82/18 |

## Examples 16-20

The processes of this group of examples were carried out by adding certain amounts (shown in the table below as a ratio with the molar amount of esters) of 50% aqueous sodium hydroxide to 21 g. of a toluene solution of isobutyl esters of the commercial C-7 acid mixture, and stirring under reflux for periods of from 1 to 6 hours. Samples were removed from the mixture, washed with water, dried, and analyzed chromatographically. The table below reports the ratio of the desired ester to the 2,2-dimethylpentanoate at each sampling interval.

| Example | Eq. of NaOH | Time | Product |
|---|---|---|---|
| 16 | 4 | 1 hr. | 71/29 |
| | | 2 | 72/28 |
| | | 3 | 74/26 |
| | | 4 | 78/22 |
| | | 5 | 87/13 |
| | | 6 | 96/4 |
| 17 | 2 | 1 hr. | 72/28 |
| | | 2 | 74/26 |
| | | 3 | 77/23 |
| | | 4 | 84/16 |
| | | 5 | 89/11 |
| | | 6 | 94/6 |
| 18 | 1 | 1 hr. | 73/27 |
| | | 2 | 77/23 |
| | | 3 | 81/19 |
| | | 4 | 87/13 |
| | | 5 | 91/9 |
| | | 6 | 93/7 |
| 19 | 8 | 1 hr. | 72/28 |
| | | 2 | 74/26 |
| | | 3 | 82/18 |
| | | 4 | 85/15 |
| | | 5 | 92/8 |
| | | 6 | 97/3 |
| 20 | 12 | 1 hr. | 70/30 |
| | | 2 | 72/28 |
| | | 3 | 74/26 |
| | | 4 | 76/24 |
| | | 5 | 78/22 |
| | | 6 | 83/17 |

## Examples 21-42

A group of hydrolyses were run under generally similar conditions. In all cases, the mixture from which the desired ester was to be purified was a toluene solution of isobutyl esters of the commercial C-7 acid mixture. The concentration of the solutions was from 355 to 425 grams of solution per mole of total esters. The base in all cases was 50% aqueous sodium hydroxide, and the hydrolyses were run under reflux. The table below reports the scale of the reaction, as the number of moles of total esters, and the amount of base, expressed as the number of equivalents of base per mole of esters. The reflux time is given in hours. The mixtures were worked up as described in Examples 12-15. The results of each example are shown as the ratio of the amount of the desired ester to the amount of 2,2-dimethylpentanoate. In some instances the percent recovery of the desired ester was calculated and is shown below.

| Example | Scale | Base | Time | Ratio | Recovery |
|---|---|---|---|---|---|
| 21 | 0.77 mole | 3.8 eq. | 4 hr. | 91/9 | |
| 22 | 0.07 | 3.9 | 4 | 80/20 | |
| 23 | 0.4 | 3.8 | 4 | 81/19 | 87% |
| 24 | 0.5 | 3.8 | 4 | 87/13 | |
| 25 | 0.5 | 3.8 | 4 | 81/19 | |
| 26 | 0.44 | 3.8 | 4 | 89/11 | 78 |
| 27 | 1.7 | 3.8 | 4 | 78/22 | 85 |
| 28 | 0.39 | 4.2 | 4 | 81/19 | 74 |
| 29 | 0.1 | 4 | 4 | 78/22 | 88 |
| 30 | 0.1 | 6 | 4 | 77/23 | 88 |
| 31 | 0.1 | 8 | 4 | 75/25 | 91 |
| 32 | 0.1 | 10 | 4 | 75/25 | 92 |
| 33 | 0.1 | 12 | 4 | 75/25 | 92 |
| 34 | 0.1 | 4 | 6 | 88/12 | 78 |
| 35 | 0.1 | 6 | 6 | 87/13 | 86 |
| 36 | 0.1 | 8 | 6 | 82/18 | 86 |
| 37 | 0.1 | 10 | 6 | 82/18 | 86 |
| 38 | 0.1 | 12 | 6 | 80/20 | 90 |
| 39 | 0.1 | 2 | 18 | 80/20 | 55 |
| 40 | 0.1 | 3 | 5.5 | 79/21 | 81 |
| 41 | 0.1 | 4 | 5 | 78/22 | 84 |
| 42 | 0.1 | 3.7 | 8 | 94/6 | 67 |

Examples 43-47

The examples reported in the table below were carried out substantially as described in the examples above, except that a phase transfer catalyst was used in each. The reflux time in all of these examples was 4 hours, and the amount of sodium hydroxide was 3 equivalents in each case, based on the total amounts of esters. The results in these examples are expressed as the ratios of the amounts of the desired ester to the 2,2-dimethylpentanoate and 2,2,3-trimethylbutanoate, respectively. In each of Examples 43, 44 and 45, the phase transfer catalyst was benzyltriethylammonium chloride, used at 0.5 mole percent, 1 mole percent and 0.5 mole percent, respectively. In Examples 46 and 47, the phase transfer catalyst was 2,2-dimethylpentanoic acid, at 0.5 mole percent and 1 mole percent, respectively. The table below reports the results.

| Example | Scale | Ratio | Recovery |
|---|---|---|---|
| 43 | 0.1 mole | 91/3.7/5.4 | 87% |
| 44 | 0.1 | 74/23/2.9 | 89 |
| 45 | 0.58 | 90/2.6/5.7 | 78 |
| 46 | 0.1 | 91/3.7/5.4 | 74 |
| 47 | 0.1 | 80/17/3.2 | 87 |

Examples 48-102

The isobutyl esters of the commercial C-7 acid mixture which were the feed stock for the examples in this group were prepared as solutions in xylene. In all of the following examples, the base was 50% sodium hydroxide, and was used at various ratios as reported in the table below. All of the reactions were carried out at the reflux temperature for different periods of time as shown below. In general, the process was as described in the examples immediately above.

The concentration of the xylene feedstock solution varied in different experiments, and is indicated below as the grams of xylene solution per mole of total esters, for each example.

In Examples 48-50 and 55-56, phase transfer catalysts were used. In Example 48, the catalyst was 1 mole percent of benzyltriethylammonium chloride, and in Examples 49-50 and 55-56 it was 2,2-dimethylpentanoic acid at 0.5%, 1%, 1% and 1%, respectively. The results of the following examples are reported in the same way as the examples immediately above.

| Example | Scale | Ester Conc. | Base | Time | Ratio | Recovery |
|---|---|---|---|---|---|---|
| 48 | 0.1 mole | 422 g./mole | 3 eq. | 4 hr. | 93/.32/6.4 | 51% |
| 49 | 0.1 | 422 | 3 | 4 | 93/0.3/6.4 | 49 |
| 50 | 0.1 | 422 | 3 | 4 | 92/0.1/7.7 | 43 |
| 51 | 0.1 | 405 | 3 | 4 | 92/1.1/6.8 | 58 |
| 52 | 0.1 | 473 | 3 | 3 | 85/9.8/5.2 | 80 |
| 53 | 0.1 | 473 | 2 | 4 | 86/9.1/5.4 | 77 |
| 54 | 0.1 | 473 | 1.5 | 4 | 87/8/5.3 | 77 |
| 55 | 2.1 | -- | 3 | 7.5 | 80/16/4.4 | 76 |
| 56 | 2.1 | -- | 3 | 7.5 | 80/16/4.6 | 76 |
| 57 | 0.1 | 382 | 3 | 4 | 92/0/7.7 | 51 |
| 58 | 0.1 | 382 | 1 | 4 | 89/4.6/6.3 | 71 |
| 59 | 0.1 | 382 | 2.5 | 3 | 92/1.3/6.9 | 63 |
| 60 | 0.1 | 382 | 2 | 3 | 91/2.5/6.6 | 66 |
| 61 | 0.1 | 382 | 1.5 | 3 | 89/4.8/613 | 70 |
| 62 | 0.1 | 382 | 1 | 3 | 84/11/5.6 | 82 |
| 63 | 0.1 | 382 | 3 | 2.5 | 93/1/5.8 | 72 |
| 64 | 0.1 | 382 | 2.5 | 2.5 | 92/1.7/6.8 | 64 |
| 65 | 0.1 | 291 | 2 | 2.5 | 90/0/9.6 | 46 |
| 66 | 0.1 | 291 | 1.5 | 2.5 | 91/0/8.9 | 56 |
| 67 | 0.1 | 291 | 1 | 2.5 | 88/3.7/7.9 | 67 |
| 68 | 0.1 | 291 | 3 | 4 | 92/0/7.6 | 39 |
| 69 | 0.1 | 291 | 3 | 2 | 90/0/10 | 35 |
| 70 | 0.1 | 291 | 2.5 | 2 | 90/0/9.5 | 47 |
| 71 | 0.1 | 291 | 2 | 2 | 90/.5/9.1 | 52 |
| 72 | 0.1 | 291 | 1.5 | 2 | 90/1.9/8.3 | 64 |
| 73 | 0.1 | 291 | 1 | 2 | 87/5.1/7.7 | 71% |
| 74 | 0.1 | 291 | 3 | 1.5 | 90/1.7/8.3 | 58 |
| 75 | 0.1 | 291 | 2.5 | 1.5 | 90/0.8/8.9 | 57 |
| 76 | 0.1 | 291 | 2 | 1.5 | 90/1.4/8.6 | 57 |
| 77 | 0.1 | 291 | 1.5 | 1.5 | 88/4.2/7.8 | 69 |

| Example | Scale | Ester Conc. | Base | Time | Ratio | Recovery |
|---|---|---|---|---|---|---|
| 78 | 0.1 mole | 291 g./mole | 1 eq. | 1.5 hr. | 80/14/6.5 | 80% |
| 79 | 0.1 | 291 | 3 | 1 | 84/8.9/7.1 | 75 |
| 80 | 0.1 | 291 | 2.5 | 1 | 87/5.8/7.5 | 70 |
| 81 | 0.1 | 291 | 2 | 1 | 78/15/6.4 | 82 |
| 82 | 0.1 | 291 | 1.5 | 1 | 83/9.9/7.0 | 77 |
| 83 | 0.1 | 291 | 1 | 1 | 78/16/6.4 | 84 |
| 84 | 0.1 | 300 | 2 | 4 | 92/.2/8.2 | 44 |
| 85 | 0.1 | 300 | 1.5 | 4 | 92/0.3/7.4 | 52 |
| 86 | 0.1 | 300 | 3 | 3 | 91/0/8.7 | 35 |
| 87 | 0.1 | 300 | 1 | 3 | 92/1.5/6.8 | 65 |
| 88 | 0.1 | 300 | 1.5 | 1 | 84/8.3/7.6 | 80 |
| 89 | 0.1 | 300 | 3 | 4 | 91/0/9.2 | 32 |
| 90 | 0.1 | 300 | 1.5 | 1 | 85/9.7/5.7 | 79 |
| 91 | 0.1 | 398 | 1.5 | 1 | 77/18/4.8 | 84 |
| 92 | 0.1 | 398 | 1.5 | 1 | 78/18/4.9 | 87 |
| 93 | 0.1 | 526 | 1.5 | 1 | 72/23/4.1 | 92 |
| 94 | 0.1 | 300 | 1.5 | 1 | 83/12/5.5 | 81 |
| 95 | 0.1 | 343 | 1.5 | 1 | 79/16/5.2 | 86 |
| 96 | 0.1 | 387 | 1.5 | 1 | 75/20/4.8 | 90 |
| 97 | 0.1 | 430 | 1.5 | 1 | 73/22/4.8 | 92 |
| 98 | 0.1 | 474 | 1.5 | 1 | 72/24/4.5 | 95 |
| 99 | 0.1 | 300 | 3 | 4 | 89/0/10.9 | 20 |
| 100 | 0.1 | 387 | 3 | 4 | 92/0.7/7.3 | 57 |
| 101 | 0.1 | 474 | 3 | 4 | 88/5.8/6.1 | 79 |
| 102 | 0.1 | 561 | 3 | 4 | 83/12/5.4 | 87 |

Examples 103-118

The isobutyl esters of the commercial C-7 acid mixture used in this group of experiments were prepared substantially as described in Preparation 1, and the esters were then isolated by distillation to assure that all unesterified carboxylic acids were removed. The mixture of esters was then dissolved in xylene to give a concentration of 300 g. of solution per mole of total esters, and the resulting ester solution was hydrolyzed substantially according to the process described in Examples 48-102.

The effect of varying amounts of carboxylic acid as catalyst was explored in these examples. The acid was 2,2-dimethylpentanoic acid, and the amount of it used is expressed in mole percent in the table below. The table also reports the reaction time in hours, and the amount of base, which was 50% sodium hydroxide, as the number of equivalents per mole of total esters. The results of the experiments are reported in the same way as the examples above.

| Example | Base | Time | Catalyst | Ratio | Recovery |
|---|---|---|---|---|---|
| 103 | 1.5 eq. | 1 hr. | 1% | 78/18/4.4 | 87% |
| 104 | 1.5 | 1 | 2 | 82/13/4.6 | 78 |
| 105 | 1.5 | 1 | 2.5 | 84/11/4.6 | 80 |
| 106 | 1.5 | 1 | 3 | 85/10/4.8 | 80 |
| 107 | 1.5 | 1 | 3.5 | 87/8.0/5.1 | 72 |
| 108 | 1.5 | 1 | 4 | 81/4.5/14 | 81 |
| 109 | 1.5 | 1 | 4.5 | 86/9.2/4.9 | 77 |
| 110 | 1.5 | 1 | 5 | 85/9.9/4.6 | 78 |
| 111 | 1.5 | 1 | 6 | 84/12/4.6 | 77 |
| 112 | 1.5 | 1 | 7 | 87/8.1/5.0 | 76 |
| 113 | 3 | 1 | 0 | 76/20/4.1 | 87 |
| 114 | 2.5 | 3.5 | 0 | 93/0.4/6.2 | 54 |
| 115 | 2.5 | 2 | 0 | 89/6.4/5.0 | 70 |
| 116 | 3 | 4 | 0 | 93/0.4/6.8 | 52 |
| 117 | 3 | 4 | 1 | 92/0/7.9 | 36 |
| 118 | 1.5 | 1 | 0 | 76/20/4.3 | 91 |

Example 119

A sample of the commercial C-7 acid mixture was esterified with 1-pentanol to obtain the amyl esters of the mixed acids, using a process substantially identical to that of Preparation 1. A total of 10.9 g. of distilled mixed esters was obtained, which was diluted with 5.4 g. of xylene, providing a concentration of about 300 g./mole. The mixture of esters was hydrolyzed with 4.26 ml. of 50% sodium hydroxide solution, for one hour at the reflux temperature, substantially as described in the examples above. The total weight of the xylene solution containing the desired ester was 12.4 g., and it was analyzed by liquid chromatography as described above, indicating that the ratio of products was 84/12/4.1, and the recovery of the desired ester was 67.0%.

Examples 120-127

The ester mixtures used in this group of examples were dissolved in toluene, and were made by the esterification of the commercial C-7 acid mixture with isobutanol in toluene by a process substantially like that of Preparation 1, except that toluene was used instead of xylene. The concentration of the ester mixture was about 552 ml./mole, and the ratio of the desired ester to the two major contaminants was about 65/32/2.5.

Each of the hydrolyses reported in these examples was carried out by adding 50 ml. of 50% aqueous

sodium hydroxide to 145 ml. of the ester solution, giving a ratio of 3.8 equivalents of base per mole of total esters. The mixture was vigorously stirred under reflux for a time shown in the table below, and the organic layer was separated, water-washed, dried by binary distillation, and analyzed to determine the ratio of the esters remaining and the yield of the desired isobutyl ester of 2-ethyl-2-methylbutanoic acid. The results are reported as they were reported in the examples above.

Some of the reaction mixtures were treated with the commercial C-7 acid mixture, or with 2,2-dimethylpentanoic acid, or both, as catalysts. Examples 121, 122 and 125 contain 2, 5 and 5 mole percent, respectively, of acid mixture, based on the amount of total ester in the reaction mixture. Examples 123, 126 and 127 contain 2 mole percent of 2,2-dimethylpentanoic acid, added as the sodium salt, and Example 124 contained 0.8 mole percent of 2,2-dimethylpentanoic acid, sodium salt, and 4 mole percent of acid mixture.

The reaction times used in each experiment and the results are shown in the table below.

| Example | Time | Ratio |
|---|---|---|
| 120 | 2 hr. | 83/17 |
| 121 | 1.5 | 83/17 |
| 122 | 2.5 | 83/17 |
| 123 | 3 | 87/13 |
| 124 | 7 | 87/13 |
| 125 | 7 | 85/15 |
| 126 | 4.5 | 88/12 |
| 127 | 6 | 93/7 |

CLAIMS

1. A process for purifying an alkyl ester of the formula

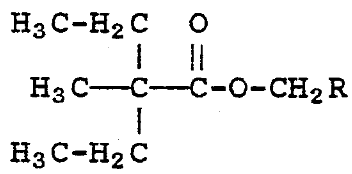

wherein

R is $C_2$-$C_5$ alkyl; provided that each carbon atom in an alkyl R group has at least one hydrogen atom bonded to it;

which ester is mixed with one or more contaminating alkyl esters of the formula

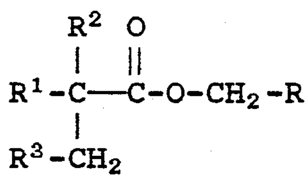

wherein

$R^1$ and $R^2$ are independently hydrogen or methyl;

$R^3$ is $C_1$-$C_5$ alkyl;

which process comprises selectively hydrolyzing the contaminating alkyl ester or esters with an alkali metal hydroxide and separating the resulting alkali metal carboxylate.

2. A process of claim 1 wherein the alkyl esters are compounds wherein R is $C_2$-$C_4$ alkyl.

3. A process of claim 2 wherein the alkyl esters are compounds wherein R is isopropyl.

4. A process of claim 1 wherein the contaminating alkyl ester or esters are compounds wherein $R^1$ and $R^2$ are methyl and $R^3$ is $C_2$-$C_3$ alkyl.

5. A process of claim 3 wherein the contaminating alkyl ester or esters are compounds wherein $R^1$ and $R^2$ are methyl and $R^3$ is $C_2$-$C_3$ alkyl.

6. A process of claim 5 wherein the alkyl ester is a compound wherein R is isopropyl.

7. A process of claim 6 wherein the contaminating alkyl ester is a compound wherein $R^1$ and $R^2$ are methyl and $R^3$ is ethyl.

8. A process of claim 1 wherein the alkali metal hydroxide is sodium hydroxide.

9. A process of claim 7 wherein the alkali metal hydroxide is sodium hydroxide.

10. A process of claim 1 wherein the process is carried out in xylene or toluene.